(19) Europäisches Patentamt
European Patent Office
Office européen des brevets

(11) Veröffentlichungsnummer : **0 381 303 B1**

(12) **EUROPÄISCHE PATENTSCHRIFT**

(45) Veröffentlichungstag der Patentschrift : **23.09.92 Patentblatt 92/39**

(51) Int. Cl.$^5$ : **A61K 31/185**

(21) Anmeldenummer : **90250012.3**

(22) Anmeldetag : **17.01.90**

(54) **Verwendung von Suramin und physiologisch verträglichen Derivaten davon, gegebenenfalls in Kombination mit anti-androgenen Mitteln zur Herstellung von Arzneimitteln zur Behandlung des humanen Prostatakarzinoms.**

(30) Priorität : **20.01.89 DE 3902021**

(43) Veröffentlichungstag der Anmeldung : **08.08.90 Patentblatt 90/32**

(45) Bekanntmachung des Hinweises auf die Patenterteilung : **23.09.92 Patentblatt 92/39**

(84) Benannte Vertragsstaaten : **AT BE CH DE DK ES FR GB GR IT LI LU NL SE**

(56) Entgegenhaltungen :
**J. CELL. BIOCHEM., Suppl. O, Band 13, Teil B, 21.-27. Januar 1989, Seite 88, Zusammenfassung Nr. E 125; C. KNABBE et al.:"Growth inhibition of human prostate cancer cells by suramin"**
**JOURNAL OF CLINICAL ONCOLOGY, Band 7, Nr. 4, April 1989, Seiten 499-508; C.A. STEIN et al.: "Suramin: An anticancer drug witha unique mechanism of action"**
**PROC. ANNU. MEET. AM. SOC. CLIN. ONCOL., Band 6, 1987, Seite 27, Zusammenfassung Nr. 100; R.V. LA ROCCA et al.: "Suramin: Anadrenocortical toxin and potential anti-neoplastic agent"**
**"The Merck Manual of Diagnosis and Therapy", 15. Ausgabe, 1987, Seiten 1649-1651, Merck & Co., Inc., Rahway, NJ, US**
**JOURNAL OF CELLULAR PHYSIOLOGY, Band 132, 1987, Seiten 143-148, Alan R. Liss, Inc.; R.J. COFFEY, Jr. et al.: "Surامininhibition of growth factor receptor binding and mitogenicity in AKR-2B cells"**
**PROC. NATL. ACAD. SCIENCE, Band 79, 1982, Seiten 1658-1662; G. TOLIS et al.: "Tumor growth inhibition in patients withprostatic carcinoma treated with luteinizing hormone-releasing hormone agonists"**

(56) Entgegenhaltungen :
**J. STEROID BIOCHEM., Band 23, 1985, Seiten 833-841; F. LABRIE et al.: "Combination therapy with flutamide and castration(LHRH agonist or orchiectomy) in advanced prostate cancer: A marked improvement in response and survival"**
**CANCER LETTERS, Band 8, 1979, Seiten 9-22, Elsevier/North-Holland Scientific Publishers Ltd; E. DE CLERCQ: "Suramin: A potentinhibitor of the reverse transcriptase of RNA tumor viruses"**

(73) Patentinhaber : **EnTec Gesellschaft für endokrinologische Technologie m.b.H.**
**Grandweg 64**
**W-2000 Hamburg 54 (DE)**

(72) Erfinder : **Voigt, Klaus-Dieter, Prof. Dr.**
**Horstlooge 22a**
**W-2000 Hamburg 67 (DE)**
Erfinder : **Knabbe, Cornelius, Dr.**
**Diekkamp 32**
**W-2000 Hamburg 67 (DE)**

(74) Vertreter : **UEXKÜLL & STOLBERG**
**Patentanwälte**
**Beselerstrasse 4**
**W-2000 Hamburg 52 (DE)**

EP 0 381 303 B1

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue Saint-Denis, 75001 PARIS

## Beschreibung

Das Prostatakarzinom (PCA) spielt eine wesentliche Rolle in der Krebsstatistik westlicher Länder. Allein in der Bundesrepublik gibt es in jedem Jahr ca. 8000 Todesfälle. Die Inzidenz mit ca. 10% aller maligner Erkrankungen bei Männern macht das PCA zum zweithäufigsten Tumor des männlichen Geschlechts. So wurden 1987 in den USA 96 000 Neuerkrankungen und 26 000 Todesfälle registriert.

Trotz intensiver Bemühungen in Forschung und klinischer Behandlung ist die altersstandardisierte Mortalitätsrate von 1950 bis 1980 beispielsweise in den USA weitgehend konstant geblieben; während in kleineren europäischen Kollektiven eine deutliche Zunahme beobachtet worden ist, die wegen der höheren Inzidenz in höherem Alter in Zusammenhang mit einer Verschiebung der Altersstruktur der Bevölkerung gesehen werden muß.

Die Androgenabhängigkeit des Prostatakarzinoms ist in der wissenschaftlichen Literatur seit den Untersuchungen von Huggins und Hodges (vgl. Cancer Res. 1, 293-297 (1941)) bekannt.

Im Anschluß an diese Arbeiten wurde eine endokrine Behandlung des Prostatakarzinoms vorgeschlagen, wobei gezeigt werden konnte, daß durch Androgenablation eine Tumorregression erreicht werden kann (vgl. Emmett et a., J. Urol. 83, 471-484 (1960)). Neben der lokalen Verdrängung des Androgens von seinen zellulären Rezeptoren durch Antiandrogene wie Flutamid oder Cyproteronacetat ist die unmittelbare Suppression zirkulierender Androgene durch chirurgische Maßnahmen wie Orchiektomie, Hypophysektomie oder Adrenalektomie oder durch die Verabreichung von Substanzen wie Diethylstilbestrol oder Aminogluthetimid daher bei der Behandlung des fortgeschrittnen Prostatakarzinoms üblich geworden.

Diese endokrine Behandlung führt in 60 bis 80% der Fälle zu einem subjektiven Erfolg. Eine objektive Verbesserung findet allerdings nur bei 20 bis 40% der Patienten statt. Zusätzlich kann eine deutliche Verängerung des symptomfreien Intervalls erreicht werden.

Allerdings führt die androgenablative Therapie nicht zu einer Heilung des Patienten. Die Mehrheit der Patienten verstirbt nach einer anfänglichen Remissionsperiode in der Regel nach dem fast obligaten Übergang des Tumors vom anfänglichen hormonabhängigen in den hormonunabhängigen Zustand.

Die Ätiologie dieses Übergangs des Tumors in den androgenunabhängigen Zustand ist gegenwärtig nicht eindeutig geklärt, wenngleich neuere Unterschungen auf eine initiale Heterogenität des Tumors hinweisen. In dem Tumor lassen sich nämlich sowohl androgenabhängige als auch androgenunabhängige Zellen nachweisen.

Auch das kürzlich beschriebene Therapieschema mit Gonadotropin-Releasing-Hormon-Analoga (GnRH-Analoga) wie z.B. Leuprolid (vgl. Schally et al., Int. J. Gynaecol. Obstet. 18, 318-324 (1989)) beruht auf einer Senkung des peripheren Androgen-Spiegels durch Hemmung der Hypophysen-Gonaden-Achse. Der Hauptvorteil dieser Therapieform liegt gegenüber der konventionellen Behandlung in geringeren Nebenwirkungen, während eine objektive Überlegenheit beispielsweise gegenüber der Therapie mit Diethylstilbestrol nicht nachweisbar war (vgl. u.a. Tolis, G. et al., Proc. Nat. Acad. Scien. 79, 1658-1662 (1982)).

Schließlich hat auch die von Labrie propagierte "komplette" Androgenablation durch Kombination von GnRH-Analoga mit nicht-steroidalen Antiandrogenen bislang weder auf experimenteller Ebene noch in klinischen Studien eine eindeutige Verbesserung gebracht (vgl. Labrie et al., J. Steroid. Biochem. 23, 833-841 (1985)).

Zusammenfassend läßt sich demgemäß feststellen, daß keiner der neueren Therapiepläne zu einer signifikanten Verbesserung der Remissions- und Überlebensrate gegenüber der klassischen endokrinologischen Therapie geführt hat.

Aufgabe der Erfindung ist es demgemäß, Mittel zur Behandlung des humanen Prostatakarzinoms zu schaffen, die gleichzeitig die androgenabhängigen und die androgenunabhängigen Zellen erfassen und damit zur Verbesserung der objektiven Remissionsrate und der Überlebenszeit des betroffenen Patienten führen.

Zur Lösung der Aufgabe wird die Verwendung von Suramin (Suramin-Natrium) und physiologisch verträglichen Derivaten davon gemäß den Ansprüchen 1 bis 4 vorgeschlagen.

Suramin-Natrium ist ein Polyanion der Formel

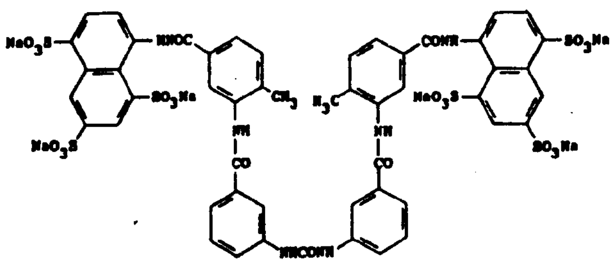

welches in der Lage ist, die Wirkung peptidischer Wachstumsfaktoren, wie beispielsweise EGF/TGF-α in Fibroblasten zu inhibieren (vgl. Coffey J.R. et al., J.Cell.Physiol. 132, 143-148 (1987)). Es ist zudem als Chemotherapeutikum gegen die Schlafkrankheit bekannt (vgl. Pharmazeutische Stoffliste, 7. Auflage, September 1987, Seiten 422, 423). Ferner wird in der DE-A-35 20 846 die Verwendung von Suramin-Natrium als Immunstimulans vorgeschlagen.

Überraschenderweise hat es sich erfindungsgemäß gezeigt, daß Suramin und physiologisch verträgliche Derivate davon in der Lage sind, die Proliferation sowohl androgenabhängiger als auch androgenunabhängiger humaner Prostatakarzinom-Zellen zu inhibieren.

Diese Substanzen hemmen sowohl androgenabhängige humane Prostatakarzinom-Zellinien (LNCaP-Zellen) als auch androgenunabhängige Prostatakarzinom-Zellinien (DU-145) (vgl. Tabelle 2).

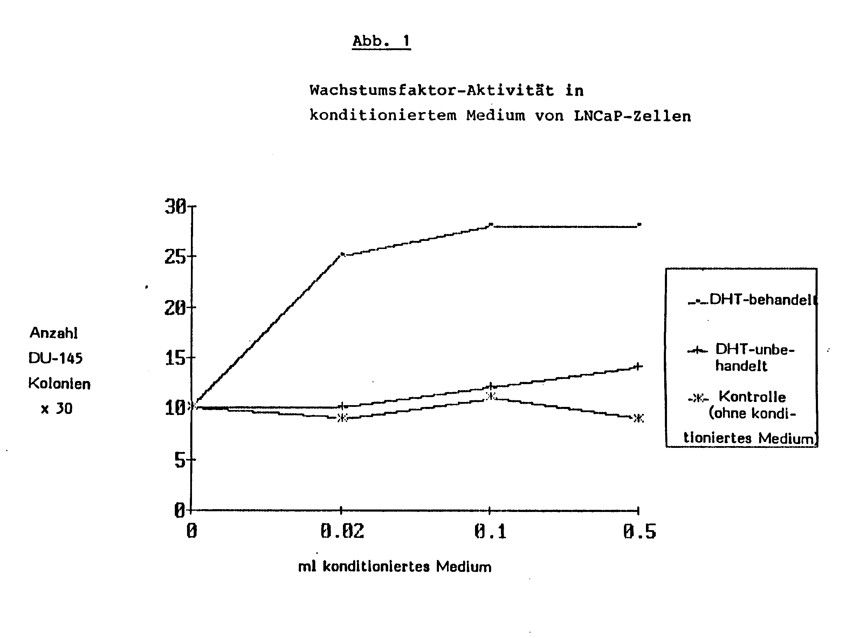

In diesem Zusammenhang konnte erfindungsgemäß erstmals gezeigt werden, daß die androgene Wachstumskontrolle des humanen Prostatakarzinoms durch die Regulation autokriner Wachstumsfaktoren vermittelt wird (Abb. 1). Ferner konnte gezeigt werden, daß diese Regulation in Gegenwart androgenabhängiger Zellen überraschenderweise auch androgenunabhängige Zellen erfaßt (vgl. Tabelle 1), und daß das Wachstum beider Zelltypen durch Suramin gehemmt werden kann (vgl. Tabelle 2).

Überraschenderweise hat es sich jedoch ferner gezeigt, daß die androgenabhängigen Karzinomzellen zusätzlich über einen Wachstumsfaktor-unabhängigen Regelkreis verfügen (vgl. Tabelle 3), der mit Suramin nicht zu beeinflussen ist. Während nämlich das Wachstum dieser Zellen in vitro in Abwesenheit von Androgenen nahezu vollständig inhibiert wird, können selbst hohe Dosierungen von Suramin deren Proliferation in Gegenwart von Androgenen (5-α-dihydro-Testosteron) nicht in diesem Umfang hemmen.

Erfindungsgemäß werden daher bei gleichzeitigem Vorliegen androgenabhängiger und androgenunabhängiger Zellen pharmazeutische Formulierungen vorgeschlagen, die eine Kombination aus Suramin oder dessen physiologisch verträglichen Derivaten und antiandrogenen Mitteln enthalten. Als letztere kommen alle mit Suramin kombinierbaren und vorzugsweise zu fixen Kombinationen verarbeitbaren Mittel in Betracht. Vorzugsweise handelt es sich um Antiandrogene im engeren Sinne, wie beispielsweise Cyproteronacetat oder Flutamid, Hemmer der hypophysären Gonadotropinsekretion, wie beispielsweise LH-RH-Agonisten oder -Antagonisten sowie Inhibitoren der steroidmetabolisierenden Enzyme, wie beispielsweise Inhibitoren der 5α-Reduktase oder Aromatase, wobei 5-α-Reduktase-Inhibitoren besonders bevorzugt sind. Es können auch mehrere antiandrogene Mittel in einer Formulierung oder Packung zusammen mit dem Suramin eingesetzt werden.

Die vorgenannten antiandrogenen Mittel können in fixer Kombination oder getrennt formuliert in einer gemeinsamen Packung mit dem Suramin oder dessen physiologisch verträglichen Derivaten vorliegen. Ihre Dosierung kann beispielsweise der üblichen und dem Fachmann bekannten Dosierung bei alleiniger Verabreichung entsprechen oder darunter liegen.

Zur Behandlung der hormonunabhängigen Form des Prostatakarzinoms können hingegen Präparate verwendet werden, die die Suramine allein enthalten, wobei Dosismengen von 1,0 bis 300, vorzugsweise 3,0 bis 30 mg/kg/Tag sowohl in den Kombinationspräparaten als auch in Suramin-Einzelpräparaten in Betracht kommen, jedoch abhängig von der klinischen Gesamtsituation des Patienten darüber oder darunter liegen können.

Die erfindungsgemäßen Präparate können lokal, oral, parenteral, intraperitoneal oder rectal verabreicht werden. Erfindungsgemäß ist die parenterale Verabreichung bevorzugt.

Bei der Herstellung der jeweiligen Formulierungen können neben Suraminen und gegebenenfalls den antiandrogenen Mitteln übliche, pharmazeutisch verträgliche Träger- und Zusatzstoffe verwendet und auf dem Fachmann bekannte Weise für die jeweils gewünschte Applikationsform zubereitet werden. Im Einzelfall kann die Bestimmung der jeweils optimalen Dosierung und Applikationsform der Wirkstoffe durch den Fachmann aufgrund seines Fachwissens leicht erfolgen.

Die Erfindung wird nachfolgend anhand von Beispielen erläutert.

In den nachfolgenden Beispielen wurden die androgenabhängige humane PCA-Zellinie LNCaP (vgl. Horoszewicz et al., Cancer Research 43, 1809-1818 (1983)), sowie die androgenunabhängige humane Prostatakarzinom-Zellinie DU-145 ATCC Nr. HTB81 verwendet und unter Standardbedingungen in DMEM-Kulturmedium angereichert mit 10% fetalem Kälberserum als Dauerkultur unterzogen.

Für die jeweiligen Wachstumsversuche wurde das Kulturmedium weitgehend von endogenen Steroiden befreit (vgl. Darbre et a., Cancer Res. 43, 349-354 (1983)).

Beispiel 1

Es wurde der Einfluß von 5-α-Dihydro-Testosteron allein sowie in Gegenwart von LNCaP-Zellen auf DU-145-Zellen untersucht.

Der Versuch wurde in einer 4-kammerigen Petrischale durchgeführt, die zuvor mit 0,1 mg/ml Poly-D-Lysin benetzt worden war.

Je 1000 DU-145 Zellen wurden in zwei Kammern ausgesät. Die Zellen in einer der Kammern wurden mit DHT in einer Endkonzentration $10^{-8}$ M in ethanolischer Lösung behandelt; während die Zellen in der zweiten Kammer zur Kontrolle nur mit dem Lösungsmittel behandelt wurden. Die Ethanol-Endkonzentration betrug in beiden Ansätzen 0,1%.

Als Wachstumsindikator wurde die nach Ablauf von 5 Tagen erreichte Anzahl der DU-145 Kolonien mit mehr als 50 Zellen gewählt, die nach Fixierung mit Methanol und Färbung mit Amidoschwarz mikroskopisch bestimmt wurde.

In einem weiteren Ansatz wurden zwei Kammern der Petrischale mit je 5 x $10^5$ LNCaP-Zellen besät und die Zellen in einer der Kammern wie oben angegeben mit DHT in ethanolischer Lösung stimuliert, während die zweite Kammer wiederum als Kontrolle diente.

Die beiden übrigen Kammern wurden wie oben angegeben jedoch ohne weitere Zusätze mit DU-145 Zellen besät.

Nach 24 Stunden wurde die Trennwand jeweils zwischen einer mit DU-135 Zellen besäten Kammer und einer mit LNCaP-Zellen besäten Kammer perforiert, so daß immer eine DU-145-Kammer mit einer LNCaP-Kammer über das Medium in Verbindung treten konnte. Die Wachstumsraten in den jeweiligen Kammern wurden erneut bestimmt.

Die Ergebnisse der beiden Ansätze sind in Tabelle 1 wiedergegeben.

**Tabelle 1**

**Wachstum von DU-145 in Kokultur mit LNCaP**

| | Behandlung mit Ethanol (=Kontrolle) | Behandlung mit DHT |
|---|---|---|
| keine LNCaP in Kokultur (=Kontrolle) | 107 | 104 |
| LNCaP in Kokultur | 136 | 213 |

Die Ergebnisse zeigen, daß DHT allein erwartungsgemäß ohne Einfluß auf die DU-145-Zellen ist, während in Gegenwart von LNCaP-Zellen ohne androgene Stimulation eine leichte Wachstumssteigerung zubeobachten ist, die sich bei androgener Stimulation erheblich steigert.

Diese Ergebnisse lassen darauf schließen, daß die unter dem Einfluß von DHT von den LNCaP-Zellen gebildete Aktivität das Wachstum auch der androgenunabhängigen Zellen anregt.

Beispiel 2

In einem weiteren Versuch wurde serumfreies, konditioniertes Medium von LNCaP-Zellen mit und ohne androgene Stimulation nach einem Verfahren, modifiziert nach Dickson et al., Endocrinology, 118, 138-142

(1986)) gewonnen.

Das konditionierte Medium wurde zur Prüfung seiner Wachstumsfaktor-Aktivität im Soft-agar-assay mit DU-145 Zellen nach dem Verfahren wie nachfolgend in Beispiel 3 erläutert untersucht.

Die Ergebnisse sind in Abb. 1 wiedergegeben. Sie bestätigen die Induktion von Wachstumsfaktor-Aktivitäten im Medium von LNCaP-Zellen durch Androgene.

Die peptidische Natur der Wachstumsfaktor-Aktivitäten wurde durch Protease- und Hitzeeinaktivierung nachgewiesen.

Beispiel 3

Die das Wachstum beider Zelltypen hemmende Wirkung von Suramin wurde im Soft-agar-assay nachgewiesen (modifiziert nach Hamburger und Salmon, Science 197, 461 (1977)).

0,8 ml einer oberen Schicht bestehend aus DMEM, 0,4% Bacto-agar, 10% fetalem Kälberserum, sowie der zu testenden Probe ($10^{-8}$) M DHZ bzw. Ethanol und/oder Suramin) und den Indikatorzellen (15.000 DU-145 oder LNCaP-Zellen) wurde in 35 mm Schalen auf eine schon ausgehärtete untere Schicht aus 1,0 ml DMEM mit 0,6% Agar und 10% fetalem Kälberserum gegeben. Die Schalen wurden über 14 Tage bei 37°C und 5% Kohlendioxid Spannung inkubiert. Als Wachstumsparameter wurden mikroskopisch Zellkolonien größer als 50 µm gezählt.

Die Ergebnisse sind in den Tabellen 2 und 3 wiedergegeben.

**Tabelle 2**

**Wachstum von LNCaP- und DU-145-Zellen**

| | -- | Suramin 100 µg/ml | Suramin 400 µg/ml |
|---|---|---|---|
| DU-145 | 400 | 170 | 25 |
| LNCaP | 625 | 150 | 50 |

**Tabelle 3**

**Wachstum von LNCaP-Zellen**

| | -- | Suramin 100 µg/ml | Suramin 400 µg/ml |
|---|---|---|---|
| Ethanol | 625 | 150 | 50 |
| + $10^{-8}$ M DHT | 1750 | 1100 | 825 |

Wie Tabelle 2 zeigt, werden sowohl LNCaP-Zellen als auch DU-145 Zellen durch Suramin bei einer Konzentration von 100 µg/ml in ihrem Wachstum deutlich gehemmt.

Im Gegensatz dazu geht aus Tabelle 3 hervor, daß selbst Suramin-Konzentrationen von 400 µg/ml in Gegenwart von DHT die Proliferation von LNCaP-Zellen nur unvollständig hemmen. Dies läßt darauf schließen, daß die Zellen über einen Suramin-resistenten Regelkreis verfügen, dessen biologische Wertigkeit und biochemische Identität noch ungeklärt sind.

Maximale Hemmung von LNCaP-Zellen ist demgemäß nur durch Gabe von Suramin in Abwesenheit von DHT (Androgenentzug) möglich.

## Patentansprüche

1. Verwendung von Suramin oder einem physiologisch verträglichen Derivat davon zur Herstellung pharmazeutischer Präparate zur Behandlung des humanen Prostatakarzinoms.

2. Verwendung nach Anspruch 1 in Verbindung mit anti-androgenen Mitteln.

3. Verwendung nach Anspruch 2, **dadurch gekennzeichnet**, daß die pharmazeutischen Präparate als fixe Kombinationen aus Suramin oder einem physiologisch verträglichen Derivat davon und einem oder mehreren antiandrogenen Mitteln vorliegen.

4. Verwendung nach Anspruch 2, **dadurch gekennzeichnet**, daß die pharmazeutischen Präparate als Packungen vorliegen, in denen das Suramin oder physiologisch verträgliche Derivat davon und das (die) anti-androgene(n) Mittel getrennt formuliert vorhanden sind.

## Claims

1. Use of suramin or a physiologically compatible derivative thereof for the manufacture of pharmaceutical preparations for the treatment of human prostatic carcinoma.

2. Use according to claim 1 in conjunction with anti-androgenic agents.

3. Use according to claim 2, characterized in that the pharmaceutical preparations are present as fixed combinations of suramin or a physiologically compatible derivative thereof and one or more anti-androgenic agents.

4. Use according to claim 2, characterized in that the pharmaceutical preparations are present as packs in which the suramin or physiologically compatible derivative thereof and the anti-androgenic agent(s) are present formulated separately.

## Revendications

1. Utilisation de suramine ou d'un dérivé physiologiquement acceptable de celle-ci pour l'obtention d'une préparation pharmaceutique pour le traitement du carcinome humain de la prostate.

2. Utilisation selon la revendication 1 , en combinaison avec des agents anti-androgènes.

3. Utilisation selon la revendication 2, caractérisée en ce que les préparations pharmaceutiques se présentent sous la forme de combinaisons fixes de suramine ou d'un dérivé physiologiquement acceptable de celle-ci et d'un ou plusieurs agents anti-androgènes.

4. Utilisation selon la revendication 2, caractérisée en ce que les préparations pharmaceutiques se présentent sous la forme de paquets, dans lesquels se trouvent la suramine ou son dérivé physiologiquement acceptable et le ou les agents anti-androgènes en formules séparées.

Abb. 1

Wachstumsfaktor-Aktivität in konditioniertem Medium von LNCaP-Zellen